# EUROPEAN PATENT APPLICATION

(11) **EP 4 642 170 A1**
(43) Date of publication of application: **29.10.2025**
(21) Application number: 25168065.8
(22) Date of filing: 02.04.2025
(51) Int. Cl.: H05H 7/02

(54) **RADIOFREQUENCY APPARATUS FOR A RADIOTHERAPY SYSTEM**

(30) Priority: 26.04.2024 CN 202410516692
(71) Applicant: Elekta Limited, Crawley, West Sussex RH10 9BL (GB)
(72) Inventor: GENG, Xingren, Crawley, RH10 9RR (GB); LIU, Ge, Crawley, RH10 9RR (GB); YAO, Fangwei, Crawley, RH10 9RR (GB); ZHANG, Xin, Crawley, RH10 9RR (GB)
(74) Representative: Thomas, Tomos Daniel

(57) **Abstract**

Disclosed herein is radiofrequency, RF, apparatus for a radiotherapy system, the RF apparatus comprising an RF power source, a three-port circulator; and a transmission waveguide arranged to be coupled to an acceleration waveguide, wherein the RF power source is arranged to transmit RF power towards the three-port circulator along a first direction and the three-port circulator is arranged to transmit RF power towards the transmission waveguide along the first direction.

## Description

This disclosure relates to apparatus, devices, systems, and approaches for radiotherapy, and in particular but without limitation to radiofrequency, RF, apparatus for a radiotherapy system.

### Background

Radiotherapy can be described as the use of ionising radiation, such as X-rays, to treat a human or animal body. Radiotherapy is commonly used to treat tumours within the body of a human or animal patient, or subject. In such treatments, ionising radiation is used to irradiate, and thus destroy or damage, cells which form part of the tumour.

Radiotherapy systems are highly complex machines having a significant number of complex interacting subsystems. Many radiotherapy systems use a beam generation subsystem based on a particle accelerator such as a linear accelerator to produce ionising radiation. Linear accelerators are powered and operated using radiofrequency apparatus, and may be mounted on or attached to a gantry for rotation to different positions about the patient, allowing the tumour to be targeted from different angles. However, it is challenging and highly technically involved to design a beam generation subsystem that is suitable for radiotherapy and enables high performance and/or versatile gantry movement.

### Summary

An invention is set out in the claims.

### Figures

Specific examples will now be described, by way of example only, with reference to the drawings of which:
Fig. 1 shows a radiotherapy device or system;
Fig. 2 shows a beam generation subsystem for a radiotherapy system;
Fig. 3 shows components of an acceleration waveguide for a linear accelerator;
Fig. 4 shows RF apparatus for a radiotherapy system;
Fig. 5 shows RF apparatus for a radiotherapy system;
Fig. 6 is a flowchart diagram which shows a method of operation of RF apparatus;
Fig. 7 shows a block diagram of one implementation of a radiotherapy system;
Fig. 8 shows a computer readable medium or, more generally, a computer program product.

### Detailed Description

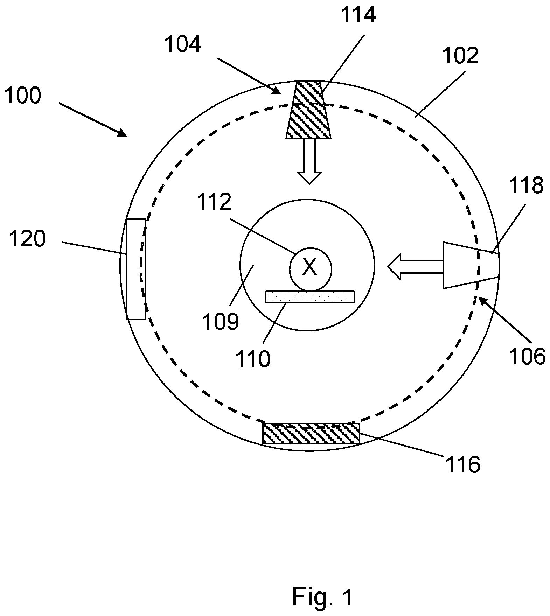

Fig. 1 shows a radiotherapy system 100, or device, suitable for delivering, and configured to deliver, a beam of radiation to a patient during radiotherapy treatment. The radiotherapy system 100 and its constituent components will be described generally for the purpose of providing useful accompanying information for the present disclosure. The radiotherapy system 100 shown in Fig. 1 is suitable for use with the disclosed methods, apparatus, and/or computer readable media.

The radiotherapy system 100 is as an image-guided radiotherapy (IGRT) machine. The radiotherapy system 100 comprises a rotatable gantry 102 to which are mounted a treatment apparatus 104 and an imaging apparatus 106. In this example, the treatment apparatus 104 and the imaging apparatus 106 are attached to the gantry, so that they are rotatable with the gantry, i.e. so that they rotate as the gantry rotates. Positioned in a treatment volume 109 of the radiotherapy system 100 is a patient support surface 110 upon which a patient 112 is positioned during radiotherapy treatment.

The patient support surface 110 is configured to move between a first position substantially outside the treatment volume 109, and a second position substantially inside the treatment volume 109. In the first position, a patient or subject can mount the patient support surface. The patient support surface 110, and patient, can then be moved inside the bore, to the second position, in order for the patient to be imaged or treated using the radiotherapy system 100. The movement of the patient support surface is effected and controlled by a patient support surface actuator, which may be described as an actuation mechanism. Together, these components may be described as a patient positioning system, which may comprise other components. The patient support surface may also be referred to as a moveable or adjustable couch or table.

Treatment apparatus 104 comprises a treatment beam source 114 and a treatment beam target 116. The treatment beam source 114 is configured to emit or direct therapeutic, or treatment, radiation, for example megavolt (MV) energy radiation, towards the treatment volume 109 and thus the patient 112. As the skilled person will appreciate, the treatment beam source 114 may comprise an electron source, a linear accelerator (linac) for accelerating electrons toward a heavy metal, e.g. tungsten, target to produce high energy photons, and a collimator configured to collimate the resulting photons and thus produce a treatment beam. Once the treatment radiation has passed from the treatment beam source 114 and through the patient 112, the treatment radiation continues towards a treatment beam target 116, where it is blocked/absorbed. The treatment beam target 116 may include an imaging panel (not shown). The treatment beam target may therefore form part of an electronic portal imaging device (EPID). EPIDs are generally known to the skilled person and will not be discussed in detail herein.

The imaging apparatus 106 comprises an imaging beam source 118 and an imaging panel 120. The imaging beam source 118 is configured to emit or direct imaging radiation, such as X-rays of kV energy, towards the patient 112. As the skilled person will appreciate, the imaging beam source 118 may be an X-ray tube or other suitable source of X-rays. The imaging beam source 119 is configured to produce kV energy radiation. Once the imaging radiation has passed from the imaging beam source 118 and through the patient 112, the imaging radiation continues towards the imaging panel 120. The imaging panel 120 may be described as a radiation detector, or a radiation intensity detector. The imaging panel 120 is configured to produce signals indicative of the intensity of radiation incident on the imaging panel 120. In use, these signals are indicative of the intensity of radiation which has passed through a patient 112. These signals may be processed to form an image of the patient 112. This process may be described as the imaging apparatus 106 and/or the imaging panel 120 capturing an image. By taking images at multiple angles around the patient it is possible to produce a 3D image of the patient, for example using tomographic reconstruction techniques.

The imaging beam source 118 may be mounted on an imaging source arm such that the imaging beam source 118 is moveable along a direction parallel to the axis of rotation of the gantry. The imaging source arm is thus configured to deploy the imaging beam source 118 to a position away from the gantry (a deployed position) for use in imaging the patient, and is configured to retract the imaging beam source 118 source to a position near to the gantry (a retracted position) for situations in which imaging is not required.

In the illustrated example, the treatment apparatus 104 and the imaging apparatus 106 are mounted on the gantry such that a treatment beam travels in a direction that is generally perpendicular to that of the imaging beam.

Because the gantry 102 is rotatable, the treatment beam can be delivered to a patient from a range of angles. Similarly, the patient can be imaged from a range of angles by the imaging apparatus 106. As the skilled person will appreciate, the gantry 102 can be rotated to any of a number of discrete angular positions relative to a patient. The treatment apparatus 104 may direct radiation toward the patient at each or a number of these discrete angular positions, according to a treatment plan. The treatment apparatus 104 may even be used to continuously irradiate a patient at all rotation angles as it is rotated by the gantry 102. The angles from which radiation is applied, and the intensity and shape of the therapeutic beam, may depend on a specific treatment plan pertaining to a given patient.

The radiotherapy apparatus 100 additionally comprises a controller (not shown). The controller comprises a computer, processor, and/or other processing device configured to control the radiotherapy apparatus 100. The controller is configured to send control signals to multiple different components of the radiotherapy apparatus 100, for example those described above and elsewhere herein. The controller is also configured to send control signals to the treatment apparatus in order to effect changes in radiotherapy treatment. The controller also collects data indicative of the performance and actions of various components of the radiotherapy apparatus 100. For example, the controller controls rotation of the gantry and records the angle to which the gantry has been rotated.

The controller may be formed by several discrete processors; for example, the controller may comprise an imaging apparatus processor, which controls the imaging apparatus 106; an treatment apparatus processor, which controls the operation of the treatment apparatus 104; and a patient support surface processor which controls the operation and actuation of the patient support surface 110. The controller is communicatively coupled to a memory, e.g. a computer readable medium, comprising computer-executable instructions which may be executed by the controller. The computer-executable, or computer-readable, instructions, may cause a processor to perform any one or more of the methods disclosed herein.

The radiotherapy apparatus 100 also comprises several other components and systems as will be understood by the skilled person. For example, in order to ensure the linac does not leak radiation, appropriate shielding is also provided.

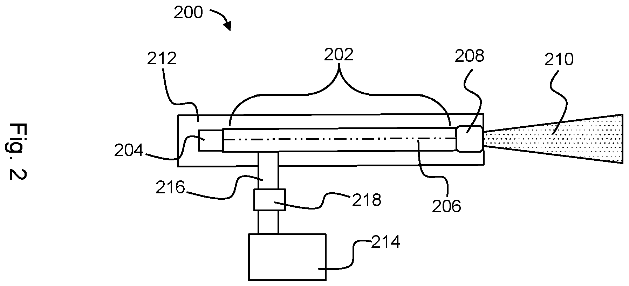

Fig. 2 shows an example beam generation subsystem 200 that will be described generally for the purpose of providing useful accompanying information for the present disclosure. For example, the beam generation subsystem 200 may be used as part of the treatment beam source 114 in the system 100 of Fig. 1. The beam generation subsystem 200 is based on a linear accelerator design, and may alternatively be referred to as a linear accelerator.

The beam generation subsystem 200 comprises an acceleration waveguide 202 and a source 204 of electrons. The source 204 of electrons may be an electron gun, for example a triode electron gun or diode electron gun.

The acceleration waveguide 202 is configured to accelerate particles, in this case electrons, along an acceleration path 206 into a target 208, in order to produce a treatment beam 210 of radiation. The acceleration path 206 is also known as the central beam axis of the acceleration waveguide 202. The acceleration waveguide 202 comprises a series of cells. In this example, each cell has substantially the same shape and dimensions, but in other examples, that may not be so. The cells may be arranged such that each cell is RF-uncoupled/independent, and in that case each cell functions as a separate resonant cavity. In other implementations, such as the example of Fig. 2, the cells may be coupled together and, in that case, the overall coupled structure may be considered to be a single resonant cavity. In such an implementation, although the coupled cells function as a single resonant cavity, individual cells may still be referred to as cavities by those skilled in the art. The acceleration path is coincident with the centre axis of the acceleration waveguide 202 and passes through an aperture at the centre of each cell. The acceleration waveguide 202, the source 204 of electrons, the cells and the target 208 are enclosed within an evacuated and vacuum-sealed casing 212 to ensure that propagation of the electrons is not impeded as they travel toward the target 208. The vacuum-sealed casing 212 is evacuated using a vacuum system to ultra-high vacuum (UHV) conditions. As the electrons are accelerated in the acceleration waveguide 202, in some embodiments the electron beam path may be controlled by a suitable arrangement of steering magnets, or steering coils (not shown), which surround the acceleration waveguide 202. The arrangement of steering magnets may comprise, for example, two sets of quadrupole magnets.

A source of RF waves, or RF power source 214, such as a magnetron or a klystron, is configured to produce and/or amplify RF waves. The RF power source 214 is coupled to the acceleration waveguide 202 via RF transmission apparatus 216, which usually comprises copper waveguide sections that can have a circular or rectangular cross section. A modulator is configured to pulse RF waves through the copper waveguide into the acceleration waveguide 202. Typically, the RF waves are input into a particular cell of the acceleration waveguide 202. The RF transmission apparatus 216 that connects the RF power source 214 to the input cell of the acceleration waveguide 202 may comprise a waveguide network and may contain an RF window which may separate a vacuum envelope from an SF6 envelope.

The RF transmission apparatus 216 is perpendicular to the acceleration waveguide 202 central beam axis 206 where it couples the power into the input cell. The RF input connecting pipe or tube is coupled with the acceleration waveguide 202 and joins the acceleration waveguide 202 at a substantially 90° angle. The RF transmission apparatus 216 may include a circulator 218.

The beam generation subsystem can operate with either a standing wave or a traveling wave configuration. In a standing wave configuration as shown, the RF power source 214 is configured to pulse RF waves into the acceleration waveguide 202, in order to set up a standing wave of varying electric field that is suitable for accelerating charged particles.

Although the RF power source 214 can operate in continuous mode, typically it operates in pulsed mode in view of the RF power levels required. An example RF wave frequency is 3 GHz, with a pulse duration in the range of microseconds and a pulse repetition rate in the range of several hundred pulses per second. The RF power source 214 may be a commercially available magnetron such as an E2V 3.1 MW magnetron, or any standard radiotherapy magnetron, operating at 3 GHz. Typically, the RF power source 214 produces each pulse at a particular phase in order to improve the stability of the standing wave within the acceleration waveguide 202. After it has been pulsed into the acceleration waveguide 202, some of the RF energy dissipates into the walls of the acceleration waveguide 202.

In an acceleration waveguide made up of coupled cells, the standing RF wave is established according to the resonant frequency of the coupled structure. An effect of coupling individually resonant cells together to form a single resonant cavity is that, due to dispersion, a band of different frequency oscillation modes comprising higher and lower order modes may be permitted within the acceleration waveguide 202 either side of the resonant frequency of the coupled structure. The frequency of the RF waves provided by the RF power source 214 determines the mode(s) that are excited in the acceleration waveguide 202.

There are also multiple modes of operation by which a standing wave at the resonant frequency can accelerate electrons within the acceleration waveguide. Electrons will accelerate or decelerate depending upon the polarity of the electric field they experience. The length of each cell in the cavity is designed such that the beam sees the same phase of the RF in each cell. The beam is synchronised such that on each oscillation the beam interacts with the positive part of the wave and is accelerated further.

In one operational mode, known as the zero mode, the electric field of the standing wave has the same polarity and magnitude in all cells at any given time. During the time that an electron takes to traverse a given cell and enter the next cell, the field makes a complete oscillation, for example from positive to negative and back to positive, such that the electron sees the same accelerating field it has just experienced, rather than a decelerating field. Alternatively, a 'π mode' may be used. Rather than the electric field being of the same polarity in each cell at a given time, adjacent cells have opposite polarities at a given time. However, the dimensions of each cell are such that during the time an electron takes to traverse a given cell, the adjacent cell experiences a half oscillation in field polarity such that the electron entering the adjacent cell experiences an accelerating field polarity rather than a decelerating one, and so on.

The source 204 of electrons, such as an electron gun, is also coupled to the acceleration waveguide 202 and is configured to inject electrons into the acceleration waveguide 202. The injection of electrons into the acceleration waveguide 202 is synchronised with the pulsing of the radiofrequency waves into the acceleration waveguide 202.

In some implementations, an upstream portion of an acceleration waveguide in a linac may be referred to as a buncher section. The buncher section may comprise one or more cells of the acceleration waveguide. Within the buncher section, the phase of the RF wave, whether a standing wave or traveling wave, decelerates some electrons to allow slower electrons to catch up, concentrating the electrons in bunches. The electrons are then free to move together in so called "packets" or "bunches" and the bunches quickly accelerate to relativistic speeds through the subsequent cells of the acceleration waveguide. The acceleration waveguide may be designed with a buncher section that is optimised to produce an electron beam with a particular energy and intensity by bunching electrons into a beam of short pulses.

RF waves may be input to the acceleration waveguide at a particular cell, or at more than one cell. In particular, RF waves may be input at a cell that is adjacent to the buncher portion of the acceleration waveguide. In the example of Fig. 2, the first two cells on the left-hand side of the acceleration waveguide 202 are the buncher and the following cells act to accelerate the electrons to relativistic speeds. Alternatively, the RF waves may be input into one or more of the cells belonging to the buncher section of the acceleration waveguide 202.

Once the electrons have been accelerated to faster energies, such as 8 MeV or 10 MeV, they may pass into a flight tube. The flight tube is connected to the acceleration waveguide by a connecting tube. The flight tube is also kept under vacuum conditions. This connecting tube or connecting structure is termed a drift tube. The drift tube also forms part of a vacuum tube along with the other components within the vacuum-sealed casing 212. The electrons may travel along a slalom path toward the heavy metal target. Whilst the electrons travel through the flight tube, an arrangement of focusing magnets act to direct and focus the beam on the target. The slalom path allows the overall length of the linac to be reduced while ensuring that the beam of accelerated electrons, which is comprised of electrons with a small spread of energies, is focused on the target.

The electrons travel toward the target 208 which may comprise, for example, tungsten, or another heavy metal. The impact of the electrons on the target 208 produces x-rays which form the treatment beam 210. When the electrons strike the target 208, x-rays are produced in a variety of directions. A primary collimator may block x-rays travelling in certain directions and pass only forward travelling x-rays to produce the treatment beam 210. The x-rays may be filtered and may pass through one or more ion chambers for dose measuring. The beam can be shaped in various ways by beam-shaping apparatus, for example by using the multi-leaf collimator 108, before it passes into the patient as part of radiotherapy treatment.

If a flight tube is used, the target is located inside the flight tube and is located at the end of the flight tube to seal the vacuum system. The flight tube also comprises a target window, which is transparent to x-rays, and which is positioned to allow the x-rays which are produced when the beam generation subsystem is in operation to pass from the evacuated flight tube through the target window and into the treatment head.

In some implementations, the electrons are accelerated within an acceleration waveguide by using a travelling wave rather than a standing wave. In this case electrons travel at the phase velocity of the travelling wave, accelerated by the longitudinal electric field component. The acceleration waveguide 202 must be designed such that the phase velocity of the traveling wave does not exceed the speed of light, otherwise no acceleration of electrons will occur. In particular, using a disk-loaded waveguide, rather than a cylindrical waveguide, reduces the phase velocity appropriately such that electrons are accelerated. For an accelerator that uses a traveling wave, in addition to an RF input, the acceleration waveguide will have an RF output configured to transfer RF energy out of the acceleration waveguide and prevent it from reflecting and establishing a standing wave. If a drift tube is used adjacent to the acceleration waveguide, the RF output may be coupled to the drift tube. As with the input transmission apparatus or waveguide, which introduces RF power to the acceleration waveguide, the output waveguide through which RF power exits the waveguide can be connected via an elbow joint or 'T-shaped' joint. RF waves pass out from the evacuated system via an RF output window which seals the vacuum envelope.

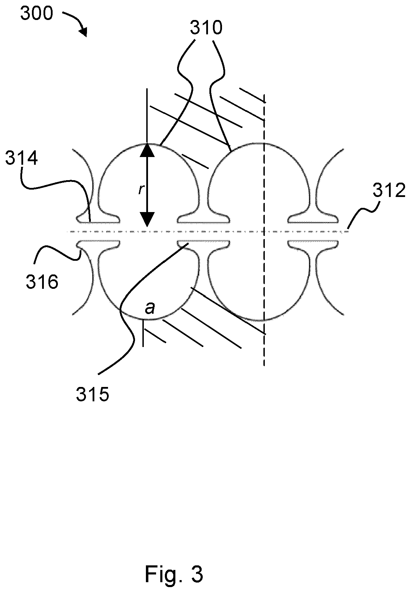

Fig. 3 shows a cross section view along the longitudinal axis of an acceleration waveguide 300 suitable for use in a particle accelerator, for example for use as the acceleration waveguide 202 of the beam generation subsystem 200 of Fig. 2, and depicts a typical multiple cell cavity. The acceleration waveguide 300 is suitable for use in a linac as shown in Fig. 1 and Fig. 2, but also could be used in other accelerators (e.g. a curved accelerator such as a synchrotron). The below examples and discussion relate to the acceleration of electrons, but the cavity can be used to accelerate any charged particle and therefore in any charged particle accelerator. For example, protons, positrons and ions can be accelerated using the techniques described herein.

Two cells 310 of a series of connected cells are shown. The cells may be coupled together. The cells are each connected along a central axis 312 by irises 314, 315. Only two cells are shown in Fig. 3, although a typical acceleration waveguide will have more. The precise number will vary, dependent on the design criteria of the accelerator. Each cell is defined in the form of a recess within a surrounding shell of a conductive material, usually copper. The acceleration waveguide 300 may be described as a disk-loaded waveguide.

In the following description, the term "longitudinal cross section" is used to define the cross section in a plane through the centre axis. The "transverse cross section" is used to define the cross section in a plane orthogonal to the centre axis. A longitudinal centre of an object is halfway down the object's longitudinal axis. For example, the longitudinal centre of a cell is the plane half way along the centre axis of that cell.

Each cell has an iris 314 connecting to the preceding cell in the sequence, and an iris 315 connecting to the next cell in the sequence. The irises and cells are centred on the centre axis. In use, the centre axis defines the electron acceleration path, the path along which electrons travel when being accelerated though the acceleration waveguide 300. Generally, cells and irises are axisymmetrical around the centre axis, forming a rounded toroid, i.e. the three-dimensional shape created by sweeping a two-dimensional shape around the axis. Although each of the cells shown in Fig. 3 has the same dimensions, with a fixed radius r and fixed length along the acceleration path 312, some waveguide designs may use coupled cells of varying dimensions, in particular cells of varying length. The length of the cell is chosen to provide a suitable length through which each electron experiences acceleration due to the oscillating RF field.

In the waveguide shown in Fig. 3, a "nose cone" 316 is formed on each end of the iris, lengthening the iris along the centre axis to protrude into the cell, for purposes of concentrating the longitudinal electric field component at the beam axis. However, some waveguides do not include a nose cone.

The cells are manufactured by welding segments of conductive material together at joining portions. The joining portions of the segments are typically in the longitudinal centre of the cell, marked a. Additionally, the plane at the longitudinal centre of each cell is a plane of symmetry. Many alternative methods may be used to make a cavity, such as brazing and diffusion bonding. One alternative to welding individual cavities together is to instead segment a larger cylindrical piece into multiple cavities.

Once a waveguide has been produced, the waveguide may be "tuned" to try to bring each cell to the correct resonance. This can be done by taking a measurement of the electric field created in a waveguide upon the application of radiofrequency energy, introducing a perturbation such as a dent into the waveguide, and then taking another measurement of the electric field to determine whether the resonance is correct. This aligns the optimum path the electrons take and minimises their interaction with the higher order modes.

Referring to the apparatuses disclosed herein, variations in design and components are possible or desirable depending upon the application requirements. For example, requirements may vary depending upon the desired type and energy of the treatment beam or depending upon the mechanical or structural design of the overall system in which the apparatus is to be used, such as the system 100 of Fig. 1.

In some implementations, the RF power source 214 may be a klystron, rather than a magnetron. Similarly, in some implementations, the RF power source 214 may be operated continuously rather than in a pulsed manner.

The beam generation subsystem 200 also comprises several other components and systems as will be understood by the skilled person. For example, in order to ensure the linac or beam generation subsystem does not leak radiation, appropriate shielding is also provided. The whole system is cooled by a water cooling system (not shown in the figures). The water cooling system may be used, in particular, to cool the acceleration waveguide 202, the target 208, and the RF power source 214.

Beam generation subsystems such as those of Figs. 2 and 3 are thus dependent upon various RF apparatus, such as the RF power source 214 and RF transmission apparatus 216, to generate and provide the electromagnetic field that is required to accelerate electrons to produce ionising radiation for treatment. For ensuring stable provision of RF power and high transmission efficiency of the acceleration waveguide, conventional RF apparatus may include further technical components, such as an automatic frequency control (AFC) system to prevent detuning of the RF power source, a water cooling system to provide stable temperature control, a four-port circulator, and use of sulphur hexafluoride (SF₆) gas within RF transmission waveguides to increase the waveguide breakdown strength limit. Conventional RF apparatus therefore has a complex structure and a large physical space requirement, which can be challenging to implement on a radiotherapy rotatable gantry.

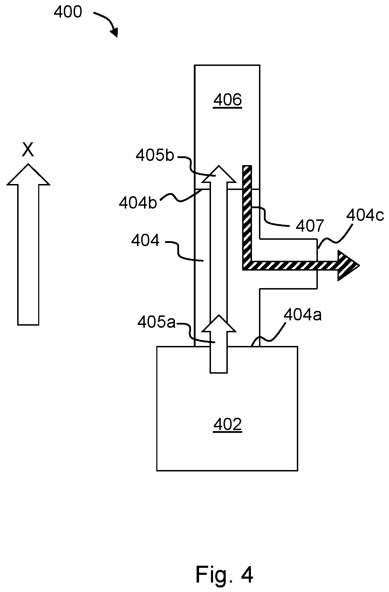

Fig. 4 shows RF apparatus 400 for a radiotherapy system which may beneficially provide a particularly compact arrangement for powering a linear accelerator, and may thus be more easily and/or stably implemented on a rotatable gantry, and may enable improved mechanical performance of the gantry, thereby improving the speed and/or accuracy at which treatment may be delivered.

The RF apparatus 400 comprises an RF power source 402. The RF power source 402 is arranged to transmit RF power towards a three-port circulator 404 along a first direction. The three-port circulator 404 is arranged to transmit RF power towards a transmission waveguide 406 along that same first direction. The transmission waveguide 406 is arranged to be coupled to an acceleration waveguide, such as those disclosed herein in relation to Figs. 2 to 3. In some examples, the transmission waveguide 406 is flexible, and may be considered to be a flexible waveguide. In some examples, a rigid waveguide may be used instead of a flexible waveguide as the transmission waveguide 406. In some examples, a waveguide that is partly rigid and partly flexible, e.g. has a rigid portion and a flexible portion, may be considered to be a flexible waveguide and may be used as the transmission waveguide 406.

The RF power source 402 may correspond to the RF power source 214 of Fig. 2. In some examples, the RF power source 402 comprises a magnetron and/or klystron. In some examples, the RF power source 402 may be arranged to generate pulsed RF power. In other examples, the RF power source 402 may be arranged to generate continuous RF power.

As will be known to the skilled person, a three-port circulator is a passive RF transmission device with three ports arranged such that RF signals may be input at any of the three ports but are only able to exit through the port that is arranged directly after the input port in a clockwise or anticlockwise (depending on the configuration of the three-port circulator) direction. In a clockwise three-port circulator, RF power that is input at a first port of the three ports will be output at a second port of the three ports that is the next port located in the clockwise direction from the first port. In the same manner, RF power may be input at the second port and will be output at a third port of the three ports that is next in the clockwise direction from the second port, and RF may be input at the third port and will be output at the first port. As will be appreciated, in an anticlockwise three-port circulator, the rotational direction of the transmission of RF power through the three ports is reversed compared with the clockwise three-port circulator.

The three-port circulator 404 of Fig. 4 is a clockwise three-port circulator arranged such that RF power may be transmitted from the RF power source 402 through a first port 404a of the three-port circulator at the interface between the RF power source 402 and the three-port circulator 404, as indicated by an arrow 405a in Fig. 4. The three-port circulator 404 is arranged such that RF power provided by the RF power source 402 will be transmitted to the transmission waveguide 406 through a second port 404b of the three-port circulator at the interface between the three-port circulator 404 and the transmission waveguide 406, as indicated by an arrow 405b in Fig. 4.

The ports of the three-port circulator 404 in the example of Fig. 4 are thus arranged such that a first port 404a is arranged to receive RF power from the RF power source 402 and the RF power will be output at the second port 404b that is clockwise from the first port 404a and located opposite the first port 404a. It will be appreciated that, in other examples, an anticlockwise direction three-port circulator may be used instead, such that the second port 404b is the next port anticlockwise from the first port 404a and is located opposite the first port 404a, with the third port being located further anticlockwise from the first port.

With the arrangement of Fig. 4, the RF power for powering the acceleration waveguide may thus be transmitted along a particular RF transmission direction (referred to elsewhere herein as "the first direction") from the RF power source 402, through the three-port circulator 404, and into the transmission waveguide 406, with each of those transmission steps being along that same direction. The transmission waveguide 406 may then transmit the RF power onwards to the acceleration waveguide. Within the RF apparatus 400, the first direction may be referred to and considered as the "main transmission line" of the RF power. If any RF power is reflected back into the second port of the three-port circulator 404, it will be transmitted out of the third port 404c of the three-port circulator 404, as indicated by an arrow 407 in Fig. 4.

It will be appreciated that, although the RF power source 402 and transmission waveguide 406 have been shown and described as each having a common interface with the three-port circulator 404, in some examples, there may instead be RF transmission apparatus between the RF power source 402 and the three-port circulator 404 and/or RF transmission apparatus between the three-port circulator 404 and the transmission waveguide 406. The RF transmission apparatus may take any suitable form, such as a waveguide and/or other RF transmission line. In general, the RF power source 402 and the transmission waveguide 406 are thus each mechanically coupled to different ports of the three-port circulator, but need not be directly mechanically coupled.

Conventionally, a larger four-port circulator is used in radiotherapy systems for performance reasons, such as to provide a particularly high isolation capability in the presence of the high power and high field working conditions of the system. However, a three-port circulator arranged to transmit RF power in a first direction as in the RF apparatus 400 disclosed herein has been found by the present inventors to be capable of supplying appropriate, stable RF power for beam generation, and, beneficially, a three-port circulator may be smaller in size than a four-port circulator, thereby enabling the overall RF apparatus size, and hence beam generation subsystem size, to be reduced. In particular, a three-port circulator arranged as in the RF apparatus 400 disclosed herein has been found to be capable of supporting suitable performance levels for a radiotherapy beam generation subsystem in terms of isolation, output dose rate, and/or output radiation energy.

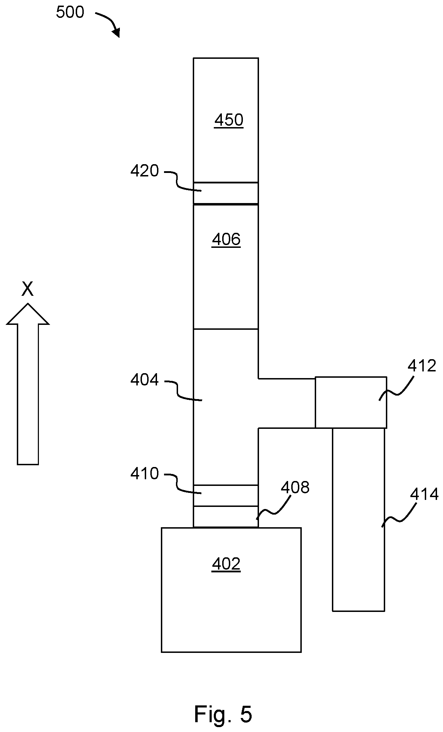

Fig. 5 shows RF apparatus 500 for a radiotherapy system that is based on the RF apparatus 400 and further comprises exemplary further components. RF apparatus 500 has the same "first direction" "main transmission line" as RF apparatus 400. The RF apparatus 500 of Fig. 5 comprises the RF power source 402, the three-port circulator 404, and the transmission waveguide 406 of the RF apparatus 400 of Fig. 4. In the example of Fig. 5, the RF power source 402 is a magnetron, but in some examples, another type of RF power source may be used, such as a klystron. RF apparatus 500 further comprises a round to rectangular waveguide transition component 408 and non-directional couplers 410 that are arranged to couple the RF power source 402 to the three-port circulator 404. It will be appreciated that, in other examples, alternative transition and/or coupler components may be used, or may not be required at all.

The RF apparatus 500 further comprises a phase shifter 412 that is coupled to the three-port circulator 404. The phase shifter 412 is arranged to adjust, or "shift", the phase of transmitted RF power. Beneficially, a phase shifter 412 can mitigate, reduce, or eliminate frequency pulling of the RF power source 402 that may occur due to improper phase of reflected RF power such as that indicated by the arrow 407 in Fig. 4. The phase shifter 412 can be particularly beneficial for mitigating any sensitivity to phase caused by the use of a three-port circulator. Advantageously, the phase-shifter 412 may be coupled to the three-port circulator 404 via transmission apparatus having a bend at an angle of 90 degrees, which makes it possible reduce the length of the system in the direction perpendicular to the first direction, and means that any increases in length due to the addition of further components are in a direction parallel to the first direction. The transmission apparatus may be, for example, a waveguide component with a bend at an angle of 90 degrees. In some examples, the phase shifter 412 is designed to cover more than 360°in the phase domain, with power reflection as low as possible.

In some examples, the phase shifter 412 is arranged to transmit RF power towards a ferrite load 414. In such examples, the three-port circulator therefore redirects reflected power away from the RF power source 402 such that the ferrite load 414 may absorb most of the reflected RF power. Furthermore, the ferrite load 414 may be coupled to the phase shifter 412. In examples in which the phase shifter 412 is coupled to the three-port circulator via transmission apparatus having a bend at an angle of 90 degrees, the ferrite load can thus be oriented with its length parallel to the first direction (either pointing in the same direction as the first direction, or the opposite direction), thereby avoiding any length increase of the RF apparatus 500 in the direction perpendicular to the first direction. In some examples, the RF apparatus 500 comprises a ferrite load 414 without a phase shifter 412, and in such examples, the three-port circulator may be arranged to direct reflected RF power towards the ferrite load 414. In some examples, a water load, or other suitable load, may be used in place of the ferrite load 414.

The RF apparatus 500 further comprises an acceleration waveguide 450 coupled to the transmission waveguide 406. The acceleration waveguide 450 may be coupled to the transmission waveguide 406 using a waveguide RF window 420. The acceleration waveguide 450 is arranged to accelerate electrons and may be in accordance with any one or more of the acceleration waveguides of the beam generation subsystems disclosed herein, such as the acceleration waveguide 202 of Fig. 2. In some examples, the acceleration waveguide 450 is arranged to accelerate electrons to an energy of at least 1 MeV. In some examples, the acceleration waveguide 450 is arranged to accelerate electrons to an energy of about 7 MeV. In some examples, the phase shifter 412 is coupled to the acceleration waveguide 450.

The RF apparatus 400, 500 may be arranged such that the RF power source 402, the three-port circulator 404, and the transmission waveguide 406 are arranged in a shared geometrical plane. For example, the RF power source 402, three-port circulator 404, and transmission waveguide 406 may be mechanically coupled to each other within a shared geometrical plane. In such arrangements, the "main transmission line" of the RF in the first direction may define one dimension of the shared geometrical plane such that the RF power source 402, three-port circulator 404, and transmission waveguide 406 are each arranged in a geometrical plane defined by the direction of the main transmission line and a direction orthogonal to the main transmission line. It will be understood that, although each component may lie in a shared geometrical plane, the components will have a finite size and height, and thus will not entirely fall within a geometrical plane; instead, a plane may be defined that intersects each of the RF power source 402, three-port circulator 404, and transmission waveguide 406. The RF apparatus 400, 500 may additionally or alternatively be arranged such that a length of the apparatus parallel to the first direction is greater than a length of the apparatus perpendicular to the first direction. For example, the RF apparatus 400, 500 may have, parallel to the first direction, an overall length within a shared geometrical plane of 2 or 3 times greater than the overall length within the shared geometrical plane in a direction perpendicular to the first direction. In some examples, the first direction is parallel to the X direction as defined in the IEC61217 standard, as shown by the arrow labelled X in Figs. 4 and 5. Correspondingly, the RF apparatus 400, 500 is thus compact in the Y-direction as defined in the IEC61217 standard. In some examples, the X and Y direction as defined in the IEC61217 standard define a shared geometrical plane of the RF power source 402, three-port circulator 404, and transmission waveguide 406. In some examples, the phase shifter 412 and/or ferrite load 414 are arranged in a shared geometrical plane with the RF power source 402, three-port circulator 404, and transmission waveguide 406.

In some examples, the transmission waveguide 406 is a rectangular waveguide. In some examples, the transmission waveguide 406 may have a length determined in accordance with an intended RF electrical length of an RF line (for example, the main RF transmission line) of the RF apparatus 400, 500. In particular, the length of the transmission waveguide 406 may be determined or chosen to ensure that the phase shift of the RF line is appropriate so that the consequent pulling effect on a magnetron or other RF power source 402 can be reduced and/or minimised, and that therefore the impact to the magnetron or other RF power source 402 performance can be reduced and/or minimised. Accordingly, in some examples, the transmission waveguide 406 has a length arranged to transmit RF power at a particular phase. The transmission waveguide 406 may be arranged to preserve the phase of RF power transmitted along the RF line or to shift the phase of RF power transmitted along the RF line, depending on the overall design of the RF apparatus 400, 500.

In addition to the use of a three-port circulator enabling a reduction in size of the RF apparatus compared with conventional radiotherapy RF power systems, the use of a flexible waveguide as the transmission waveguide 406 further enables the RF apparatus to be formed as a compact structure with reduced size. The flexibility of the flexible waveguide further beneficially assists with alignment of the RF apparatus 400, 500 with the acceleration waveguide and on a rotatable gantry, and can allow the RF apparatus 400, 500 to fit the rotatable gantry more closely, reducing the overall size of the system and providing mechanical versatility. The use of a three-port circulator and a flexible waveguide for providing RF power to an acceleration waveguide thus enables RF apparatus 400, 500 to be implemented in a particularly compact and stable manner on a radiotherapy gantry.

The flexible waveguide further enables fast installation and adjustment of the RF apparatus disclosed herein, as the flexible waveguide can adjust itself to alignment requirements of any flanges that may be used, reducing the effects of misalignment and stresses caused by mechanical connections. In some examples, the flexible waveguide is bendable, which may enable the RF apparatus 400, 500 to be provided to a rotatable gantry with a better fit. In some examples, the transmission waveguide has a length of between 135 mm and 145 mm in a direction parallel to the first direction, which allows for low loss RF transmission, thereby enabling the RF apparatus 400, 500 to provide particularly low insert loss and stable RF power to the acceleration waveguide. In some examples, the transmission waveguide has a length of 138 mm. In some examples, the transmission waveguide has a length which corresponds to a whole wavelength of the RF power with which the system is to be powered, such as a length of 138 mm corresponding to a whole wavelength of a 2998 MHz RF wave.

By making use of the compact arrangement of RF apparatus 400, 500 of Figs. 4 and 5, the RF apparatus may be mounted to a rotatable gantry and may be tilted out of the plane of rotation of the gantry to a tilted position Thus, the RF apparatus disclosed herein enables the beam generation subsystem to be manoeuvred in a more versatile manner on a rotatable gantry..

Accordingly, disclosed herein is a radiotherapy system comprising an RF apparatus in accordance with the examples of Fig. 4 or Fig. 5, and a rotatable gantry, wherein the RF apparatus is mounted to the rotatable gantry and is arranged to be tiltable with respect to a plane within which the rotatable gantry is rotatable.

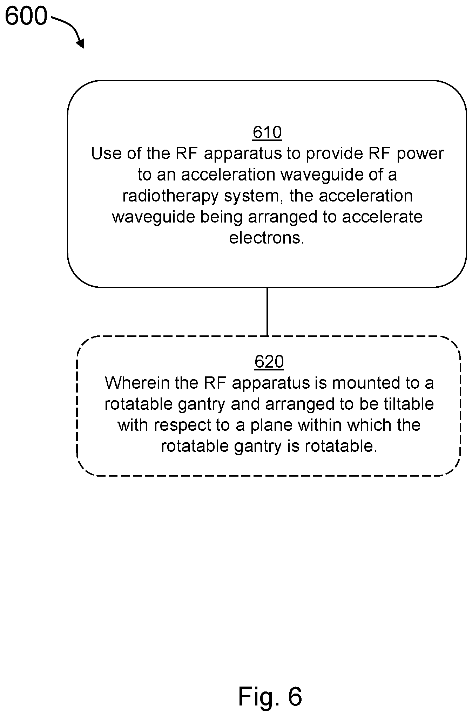

Fig. 6 is a flowchart diagram which shows a method 600 of operation of RF apparatus for a radiotherapy system. At a first block 610, the method comprises use of the RF apparatus 400 of Fig. 4 or the RF apparatus 500 of Fig. 5 to provide RF power to an acceleration waveguide of a radiotherapy system, the acceleration waveguide being arranged to accelerate electrons. At a second block 620, optionally, the RF apparatus 400, 500 is mounted to a rotatable gantry and arranged to be tiltable with respect to a plane within which the rotatable gantry is rotatable. The method 600 may further comprise, for example, tilting the RF apparatus 400, 500 with respect to a plane within which the rotatable gantry is rotatable. The method 600 may further comprise, for example, providing electrons to the acceleration waveguide, and accelerating the electrons into a target in order to generate x-rays.

A computer-based system may be used for controlling or operating various parts of the systems, devices, methods and apparatuses disclosed herein. The computer-based system can be implemented in software, firmware and/or hardware and may comprise a computer-readable medium containing instructions that, when executed by a processor, cause the system to perform any of the methods described herein.

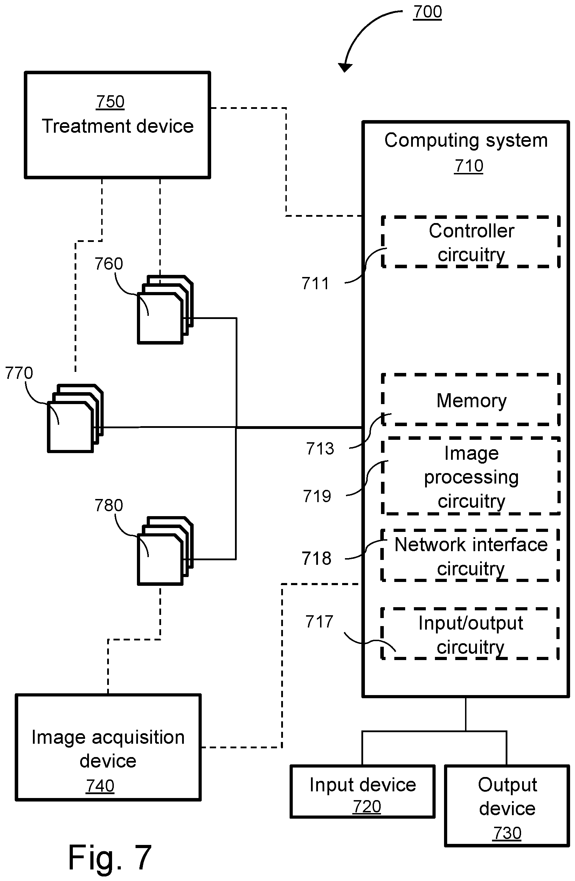

Fig. 7 shows a block diagram of one implementation of a radiotherapy system 700. The radiotherapy system 700 comprises a computing system 710 within which a set of instructions, for causing the computing system 710 to perform any one or more of the methods discussed herein, may be executed.

The computing system 710 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 710 includes controller circuitry 711 and a memory 713 (e.g., read-only memory (ROM), flash memory, dynamic random access memory (DRAM) such as synchronous DRAM (SDRAM) or Rambus DRAM (RDRAM), etc.). The memory 713 may comprise a static memory (e.g., flash memory, static random access memory (SRAM), etc.), and/or a secondary memory (e.g., a data storage device), which communicate with each other via a bus (not shown).

Controller circuitry 711 represents one or more general-purpose processors such as a microprocessor, central processing unit, accelerated processing units, or the like. More particularly, the controller circuitry 711 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 711 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 711 is configured to execute the processing logic for performing the operations and steps discussed herein.

The computing system 710 may further include a network interface circuitry 718. The computing system 710 may be communicatively coupled to an input device 720 and/or an output device 730, via input/output circuitry 717. In some implementations, the input device 720 and/or the output device 730 may be elements of the computing system 710. The input device 720 may include an alphanumeric input device (e.g., a keyboard or touchscreen), a cursor control device (e.g., a mouse or touchscreen), an audio device such as a microphone, and/or a haptic input device. The output device 730 may include an audio device such as a speaker, a video display unit (e.g., a liquid crystal display (LCD) or a cathode ray tube (CRT)), and/or a haptic output device. In some implementations, the input device 720 and the output device 730 may be provided as a single device, or as separate devices.

In some implementations, the computing system 710 may comprise image processing circuitry 719. Image processing circuitry 719 may be configured to process image data 780 (e.g. images, or imaging data), such as medical images obtained from one or more imaging data sources, a treatment device 750 and/or an image acquisition device 740. Image processing circuitry 719 may be configured to process, or pre-process, image data. For example, image processing circuitry 719 may convert received image data into a particular format, size, resolution or the like. In some implementations, image processing circuitry 719 may be combined with controller circuitry 711.

In some implementations, the radiotherapy system 700 may further comprise an image acquisition device 740 and/or a treatment device 750, such as those disclosed herein in the example of Fig. 1. The image acquisition device 740 and the treatment device 750 may be provided as a single device. In some implementations, treatment device 750 is configured to perform imaging, for example in addition to providing treatment and/or during treatment. The treatment device 750 comprises the main radiation delivery components of the radiotherapy system, such as the beam generation subsystems and linear accelerator components disclosed herein.

Image acquisition device 740 may be configured to perform positron emission tomography (PET), computed tomography (CT), and magnetic resonance imaging (MRI).

Image acquisition device 740 may be configured to output image data 780, which may be accessed by computing system 710. Treatment device 750 may be configured to output treatment data 760, which may be accessed by computing system 710.

Computing system 710 may be configured to access or obtain treatment data 760, planning data 770 and/or image data 780. Treatment data 760 may be obtained from an internal data source (e.g. from memory 713) or from an external data source, such as treatment device 750 or an external database. Planning data 770 may be obtained from memory 713 and/or from an external source, such as a planning database. Planning data 770 may comprise information obtained from one or more of the image acquisition device 740 and the treatment device 750.

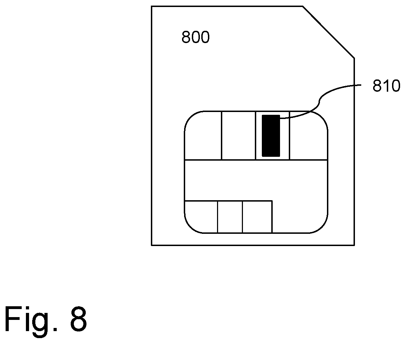

The various methods described above may be implemented by a computer program. Fig. 8 shows a computer readable medium or, more generally, a computer program product. The computer program may include computer code (e.g. instructions) 810 arranged to instruct a computer to perform the functions of one or more of the various methods described above. The steps of the methods described above may be performed in any suitable order. For example, block 620 of method 600 may be performed before, after, simultaneously or substantially simultaneously with block 610. The computer program and/or the code 810 for performing such methods may be provided to an apparatus, such as a computer, on one or more computer readable media or, more generally, a computer program product 800)), depicted in Fig. 8. The computer readable media may be transitory or non-transitory. The one or more computer readable media 800 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 810 may also reside, completely or at least partially, within the memory 713 and/or within the controller circuitry 711 during execution thereof by the computing system 710, the memory 713 and the controller circuitry 711 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

The term "apparatus" as used herein may refer to either a single apparatus or plural apparatus and should not be understood as being particularly limited to either a single discrete apparatus or a plurality of discrete apparatus unless a particular apparatus is further described as such.

Those skilled in the art will recognise that a wide variety of modifications, alterations, and combinations can be made with respect to the above described examples without departing from the scope of the disclosed concepts, and that such modifications, alterations, and combinations are to be viewed as being within the ambit of the disclosed concepts. Those skilled in the art will also recognise that the scope of the invention is not limited by the examples described herein but is instead defined by the appended claims.

## Claims

1. Radiofrequency, RF, apparatus for a radiotherapy system, the RF apparatus comprising:
an RF power source;
a three-port circulator; and
a transmission waveguide arranged to be coupled to an acceleration waveguide,
wherein the RF power source is arranged to transmit RF power towards the three-port circulator along a first direction and the three-port circulator is arranged to transmit RF power towards the transmission waveguide along the first direction.

2. The RF apparatus of claim 1, further comprising a phase shifter, wherein the phase shifter is coupled to the three-port circulator.

3. The RF apparatus of claim 2, wherein the phase shifter is coupled to the three-port circulator via transmission apparatus having a bend at an angle of 90 degrees.

4. The RF apparatus of claim 2 or claim 3, wherein the phase shifter is arranged to transmit RF power towards a ferrite load.

5. The RF apparatus of any preceding claim, wherein the transmission waveguide is a flexible waveguide.

6. The RF apparatus of claim 5, wherein the transmission waveguide is bendable.

7. The RF apparatus of any preceding claim, wherein the RF power source, the three-port circulator, and the transmission waveguide are arranged in a shared geometrical plane.

8. The RF apparatus of any preceding claim, wherein a length of the RF apparatus parallel to the first direction is greater than a length of the RF apparatus perpendicular to the first direction.

9. The RF apparatus of any preceding claim, wherein the first direction is parallel to the X direction as defined in the IEC61217 standard.

10. The RF apparatus of any preceding claim, further comprising an acceleration waveguide coupled to the transmission waveguide, wherein the acceleration waveguide is arranged to accelerate electrons.

11. The RF apparatus of claim 10, wherein the acceleration waveguide is arranged to accelerate electrons to an energy of at least 1 MeV, optionally wherein the acceleration waveguide is arranged to accelerate electrons to an energy of about 7 MeV.

12. A radiotherapy system comprising the RF apparatus of any preceding claim and a rotatable gantry, wherein the RF apparatus is mounted to the rotatable gantry and is arranged to be tiltable with respect to a plane within which the rotatable gantry is rotatable.

13. A method of operating a radiotherapy system, the method comprising use of the RF apparatus of any of claims 1 to 11 to provide RF power to an acceleration waveguide of the radiotherapy system, the acceleration waveguide being arranged to accelerate electrons.

14. The method of claim 13, wherein the radiotherapy system comprises a rotatable gantry, and wherein the RF apparatus is mounted to the rotatable gantry and arranged to be tiltable with respect to a plane within which the rotatable gantry is rotatable.

15. A computer-readable medium containing instructions that, when executed by a processor, cause the method of claim 13 or claim 14 to be performed.
